# EUROPEAN PATENT SPECIFICATION

(11) **EP 4 613 315 B1**
(45) Date of publication and mention of the grant of the patent: **26.08.2026**
(21) Application number: 25157659.1
(22) Date of filing: 13.02.2025
(51) Int. Cl.: A61M 16/18

(54) **ENGAGEMENT MECHANISM FOR VAPORIZER IN ANESTHESIA MACHINE**
EINRASTMECHANISMUS FÜR VERDAMPFER IN EINER ANÄSTHESIEMASCHINE
MÉCANISME D'ENGAGEMENT POUR VAPORISATEUR DANS UNE MACHINE D'ANESTHÉSIE

(30) Priority: 08.03.2024 US 202418600192
(43) Date of publication of application: 10.09.2025
(73) Proprietor: GE Precision Healthcare LLC, Waukesha, Wisconsin 53188 (US)
(72) Inventor: DING, Chao, Wuxi, 214028 (CN); LÓNYAY, János G., Madison, 53718 (US); BAI, Vincent, Wuxi, 214028 (CN); LIU, Albert, Shanghai, 200235 (CN); FANG, Hui, Wuxi, 214028 (CN)
(74) Representative: Fennell, Gareth Charles

(56) References cited:
- WO-A1-2021/134368

## Description

### BACKGROUND

Generally, this application relates to the engagement mechanism for a vaporizer when it is inserted and removed from an interface in an anesthesia machine.
WO 2021/134368 discloses an anesthesia ventilation system and an anesthesia evaporator. The system comprises: the anesthesia evaporator ; an evaporator connecting base; an in-place detection device which detects whether the anesthesia evaporator is connected to the evaporator connecting base; a gas inlet interface and a gas outlet interface, which are provided on a fresh gas branch and are connected to the anesthesia evaporator when the anesthesia evaporator is connected to the evaporator connecting base; and a controller which supplies gas to the anesthesia evaporator by means of the gas inlet interface and receives anesthetic gas output by the anesthesia evaporator by means of the gas outlet interface when the in-place detection device detects that the anesthesia evaporator is connected to the evaporator connecting base. The gas is supplied to the anesthesia evaporator by means of the gas inlet interface and the anesthetic gas output by the anesthesia evaporator is received by means of the gas outlet interface only when the in-place detection device detects that the anesthesia evaporator is connected to the evaporator connecting base. The case that the anesthesia evaporator can work when being not reliably connected to the evaporator connecting base can be prevented from affecting a doctor to normally use.

### SUMMARY

According to embodiments, an anesthesia machine for engaging a vaporizer with an interface of the anesthesia machine is provided, wherein the vaporizer includes an upper surface with a shelf and an recess, wherein the anesthesia machine includes: a button configured to receive an engagement force from a user and responsively move inwardly; a wedge including at least one upwardly-facing sloped region and at least one downwardly-facing sloped region; and a latch including an engagement portion configured to engage with the upper surface of the vaporizer, and including at least one laterally-projecting portion configured to engage with the at least one upwardly-facing sloped region and the at least one downwardly-facing sloped region of the wedge, wherein the engagement portion of the latch is configured to be forced upwardly when the vaporizer is being inserted into the interface of the anesthesia machine in response to the engagement portion of the latch engaging with the shelf of the upper surface of the vaporizer, and wherein the at least one laterally-projecting portion of the latch is configured to engage with the at least one downwardly-facing sloped region of the wedge when the engagement portion of the latch engages with the shelf of the upper surface of the vaporizer, thereby causing the wedge and the button to move inwardly, wherein the engagement portion of the latch is configured to be received by the recess of the upper surface of the vaporizer, thereby causing the wedge and the button to move outwardly, and wherein the button is configured to receive the engagement force while the engagement portion of the latch is received in the recess in the upper surface of the vaporizer, thereby causing the at least one upwardly-facing sloped region of the wedge to engage with the at least one laterally-projecting portion of the latch, thereby causing the engagement portion of the latch to be lifted upwardly out of the recess in the upper surface of the vaporizer. The anesthesia machine may further include a button return spring interposed between the button and a housing of the anesthesia machine, wherein the button return spring causes the button and the wedge to move outwardly. The anesthesia machine may further include a latch compression spring configured to exert a force on the latch thereby causing the engagement portion of the latch to exert a force downwardly. The at least one upwardly-facing sloped region of the wedge may include a first upwardly-facing sloped region and a second upwardly-facing sloped region; the at least one downwardly-facing sloped region of the wedge may include a first downwardly-facing sloped region and a second downwardly-facing sloped region; the at least one laterally-projecting portion of the latch may include a first laterally-projecting portion and a second laterally-projecting portion; wherein the first laterally-projecting portion of the latch may be configured to engage with the first upwardly-facing sloped region of the wedge and the first downwardly-facing sloped region of the wedge; and wherein the second laterally-projecting portion of the latch may be configured to engage with the second upwardly-facing sloped region of the wedge and the second downwardly-facing sloped region of the wedge. A slope of the at least one downwardly-facing sloped region may be greater than a slope of the at least one upwardly-facing sloped region. The vaporizer may include a vaporizer configured to contain a gas. The latch may include a pivot configured to be rotatably moved with respect to the housing, such that the latch is configured to rotate around the pivot. The wedge may include a hook including one of the at least one downwardly-facing sloped region and one of the at least one upwardly-facing sloped region. The wedge may include a plurality of rails configured to be received by a corresponding plurality of tracks in the housing of the anesthesia machine, and wherein the wedge further may include a plurality of spring arms configured to secure the wedge to the plurality of tracks. The interface of the anesthesia machine may include a housing having an outer surface, wherein when an outer surface of the button is flush with the outer surface of the housing, it indicates that the vaporizer has been fully received by the interface, and wherein when the outer surface of the button is not flush with the outer surface of the housing, it indicates that the vaporizer has not been fully received by the interface.

According to embodiments, an anesthesia machine includes an interface for receiving a vaporizer, the anesthesia machine further includes: a housing having an outer surface; a button having an outer surface, and configured to receive an engagement force from a user; and a latch including an engagement portion, and configured to selectively secure the vaporizer to the anesthesia machine via the engagement portion according to a position of the button, wherein a positional relationship between the outer surface of the button with the outer surface of the housing indicates whether or not the vaporizer is correctly received by the interface of the anesthesia machine. When the outer surface of the button is not in a predetermined position with the outer surface of the housing, this may indicate that the vaporizer has not been fully received by the anesthesia machine. The predetermined position may be when the outer surface of the button is substantially flush with the outer surface of the housing. The anesthesia machine may further include a wedge configured to move with the button, wherein the wedge may be configured to interact with the latch to force the engagement portion of the latch away from the vaporizer when the button is forced inwardly. The wedge may include at least one upwardly-facing sloped region configured to interact with the latch to force the engagement portion of the latch away from the vaporizer. The wedge may be configured to interact with the latch and to pull the button inwardly when the engagement portion of the latch is being forced by the vaporizer. The wedge may include at least one downwardly-facing sloped region configured to interact with the latch to cause the wedge to pull the button inwardly when the engagement portion of the latch is being forced by the vaporizer.

According to embodiments, a method for inserting a vaporizer into an interface of an anesthesia machine includes: partially receiving the vaporizer in the interface, thereby causing a button of the anesthesia machine to not be in a predetermined position; and fully receiving the vaporizer in the interface, thereby causing the button of the anesthesia machine to be in a predetermined position. When the vaporizer is fully received by the interface, the vaporizer may be engaged with the anesthesia machine. When the button receives a force from a user, the vaporizer may be disengaged with the anesthesia machine.

The invention is defined by the appended claims.

### BRIEF DESCRIPTION OF SEVERAL VIEWS OF THE DRAWINGS

FIG. 1 illustrates an anesthesia machine and two removable vaporizers, according to embodiments.
FIG. 2A illustrates a portion of an anesthesia machine and two removable vaporizers, with the vaporizers fully inserted into the anesthesia machine, according to embodiments.
FIG. 2B illustrates a portion of an anesthesia machine and one removable vaporizer, where the anesthesia machine has an empty interface for receiving another removable vaporizer, according to embodiments.
FIG. 2C illustrates a portion of an anesthesia machine and two removable vaporizers, where one of the removable vaporizers is not fully inserted into an interface of the anesthesia machine, according to embodiments.
FIG. 3A illustrates a top perspective view of the engagement mechanism for engaging a removable vaporizer in an interface of an anesthesia machine, according to embodiments.
FIG. 3B illustrates a bottom perspective view of the engagement mechanism for engaging a removable vaporizer in an interface of an anesthesia machine, according to embodiments.
FIG. 3C illustrates an exploded view of the engagement mechanism for engaging a removable vaporizer in an interface of an anesthesia machine, according to embodiments.
FIG. 3D illustrates an exploded view of a portion of the engagement mechanism for engaging a removable vaporizer in an interface of an anesthesia machine, according to embodiments
FIG. 4A illustrates a cross-sectional view of a portion of an anesthesia machine including the engagement mechanism, and a portion of a removable vaporizer, where the removable vaporizer is fully inserted into an interface of the anesthesia machine, according to embodiments.
FIG. 4B illustrates a cross-sectional view of a portion of an anesthesia machine including the engagement mechanism, and a portion of a removable vaporizer, where the removable vaporizer is partially inserted into an interface of the anesthesia machine, according to embodiments.
FIG. 4C illustrates a cross-sectional view of a portion of an anesthesia machine including the engagement mechanism, and a portion of a removable vaporizer, where the removable vaporizer is fully inserted into an interface of the anesthesia machine, and where the engagement mechanism has been engaged by a user to disengage the engagement mechanism from the removable vaporizer, according to embodiments.
FIG. 5A illustrates a cross-sectional view with perspective of a portion of an anesthesia machine including the engagement mechanism, and a portion of a removable vaporizer, where the removable vaporizer is fully inserted into an interface of the anesthesia machine, according to embodiments.
FIG. 5B illustrates a cross-sectional view with perspective of a portion of an anesthesia machine including a portion of the engagement mechanism, according to embodiments.
FIG. 6 is a flowchart of a method for engaging and disengaging a vaporizer from an anesthesia machine, according to embodiments.

The foregoing summary, as well as the following detailed description of certain techniques of the present application, will be better understood when read in conjunction with the appended drawings. For the purposes of illustration, certain techniques are shown in the drawings. It should be understood, however, that the claims are not limited to the arrangements and instrumentality shown in the attached drawings. Furthermore, the appearance shown in the drawings is one of many ornamental appearances that can be employed to achieve the stated functions of the system.

### DETAILED DESCRIPTION

Embodiments of an anesthesia machine are disclosed herein that receives a removable vaporizer. The anesthesia machine includes an engagement mechanism, embodiments of which are described herein. The engagement mechanism may lock the vaporizer automatically once the vaporizer is inserted, and the engagement mechanism may unlock the vaporizer once a button is pressed. The engagement mechanism may further provide a safety/warning indicator as well, in case the vaporizer is unlatched or not fully received at the interface of the anesthesia machine. This indication may occur even when no power is supplied to the anesthesia machine and/or vaporizer, as the engagement mechanism may be implemented mechanically only. The button of the engagement mechanism may have a predetermined position when the vaporizer is fully inserted into the interface of the anesthesia machine. If the button is not in the predetermined position, then that may indicate that the vaporizer is not fully inserted.

According to embodiments, when the vaporizer is inserted into the interface of the anesthesia machine, a latch in the engagement mechanism may be lifted and then may drop into a recess on the outer surface of the vaporizer. When the latch is lifted up, the button may not be in the predetermined position. When the latch drops into the recess on the outer surface of the vaporizer, the button may be placed in the predetermined position. Further, when the button is pressed, the latch may be lifted out of the recess on the outer surface of the vaporizer, thereby disengaging the vaporizer and allowing for its removal. The button may return to the predetermined position after vaporizer is removed from the anesthesia machine by the user.

According to embodiments, the engagement mechanism includes a wedge having sloped regions. a latch, and a button. The wedge may interact with the latch and the button. Certain sloped region(s) in the wedge cause the latch to be removed from a recess in the vaporizer when a user presses the button. Removing the latch from the recess may disengage the vaporizer from the anesthesia machine, such that a user may remove the vaporizer. When the latch is forced away from the vaporizer, this may cause the wedge and the button to move away from a predetermined position. When the button is not in the predetermined position, this may serve as an alert that the vaporizer has not been fully received by the interface of the anesthesia machine.

While embodiments described herein primarily relate to an anesthesia machine and a vaporizer, the engagement mechanism may be incorporated into other types of devices or systems other than an anesthesia machine. Further, instead of a vaporizer, the engagement mechanism may selectively engage or disengage another kind of mechanical component. Thus, the disclosure is not limited to the specific combination of anesthesia machines and vaporizers.

Aspects of the present disclosure have the technical effect of engaging and disengaging a vaporizer from an anesthesia machine. Various embodiments have the technical effect of indicating to a user that the vaporizer is not fully received by an interface of the anesthesia machine. Various embodiments have the technical effect of providing the disclosed functionality without the need for electric power.

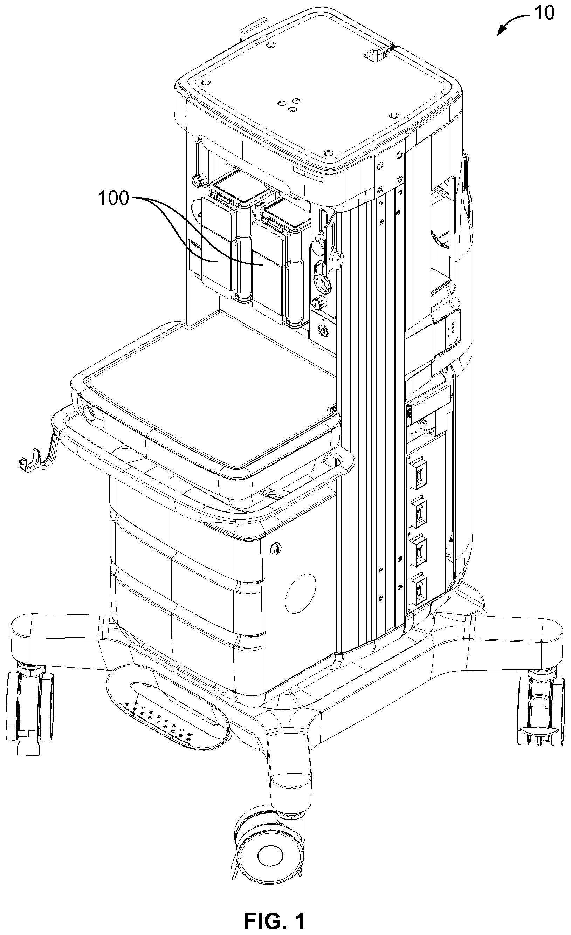

FIG. 1 illustrates an anesthesia machine 10 and two removable vaporizers 100, according to embodiments. The anesthesia machine 10 is used by a clinician to deliver anesthetic gas to a patient. Exemplary embodiments of the anesthesia machine 10 are disclosed in U.S. Pat. Nos. 11,318,274 and 11,439,789. Exemplary vaporizers 100 may be devices that induce and control vaporization of a volatile anesthetic agent from a liquid form. Exemplary embodiments of a vaporizer 100 are disclosed in U.S. Pat. Nos. 11,318,274 and 11,439,789.

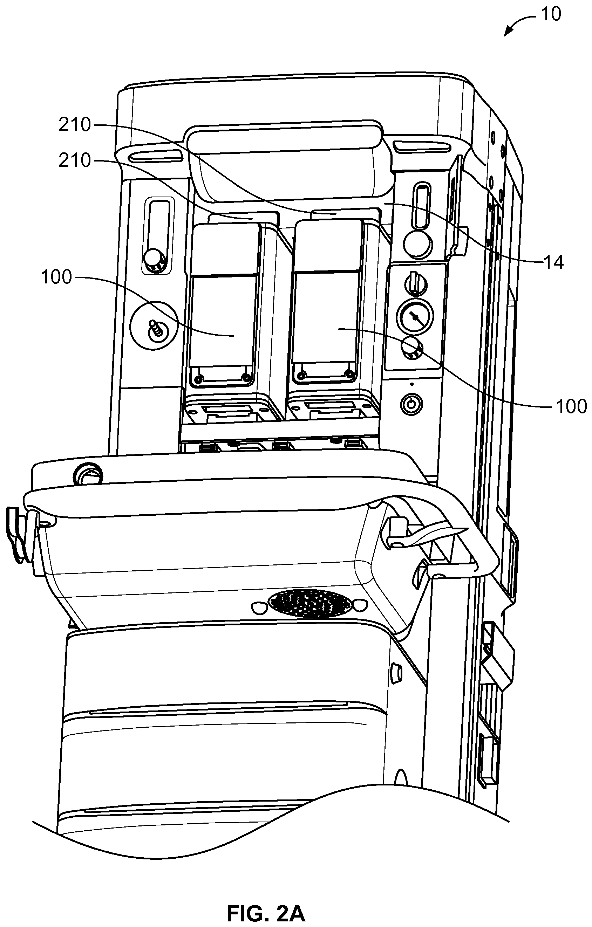

FIG. 2A illustrates a portion of the anesthesia machine 10 and two removable vaporizers 100, with the vaporizers 100 fully received by corresponding interfaces 11 of the anesthesia machine 10, according to embodiments. FIG. 2A further shows two buttons 210 from two corresponding engagement mechanisms 200 (not fully shown). The buttons 210 are configured to receive a disengagement force from a user to disengage the corresponding vaporizers 100 from the anesthesia machine 10, such that the corresponding vaporizers 100 can be removed. The interfaces 11 of the anesthesia machine 10 are not visible in FIG. 2A, because the vaporizers 100 are fully received. The buttons 210 are in a predetermined position indicating that the corresponding vaporizers 100 are fully received or engaged with the anesthesia machine 10. In the depicted embodiment, the predetermined position is when an outer surface of a given button 210 is substantially flush with the housing 14 (e.g., surrounding housing) of the anesthesia machine 10.

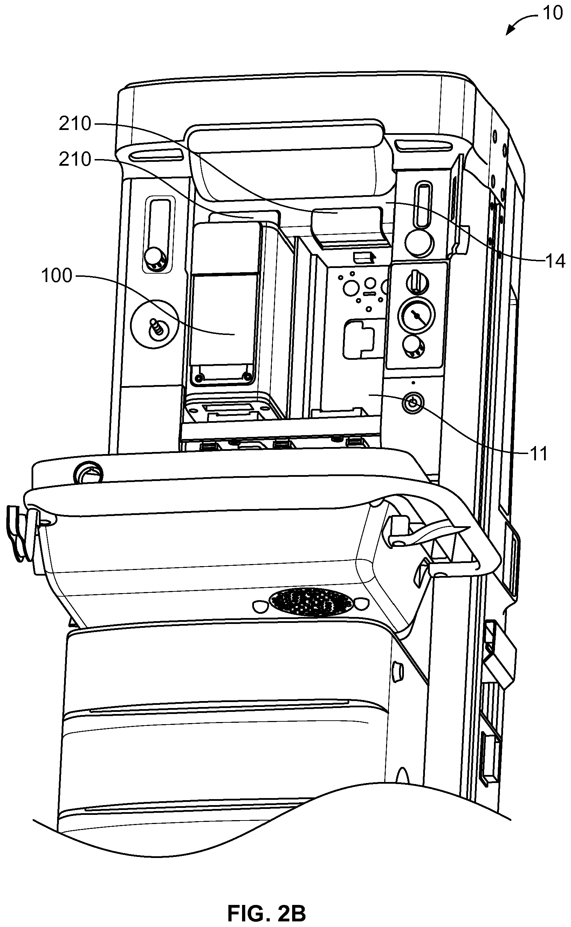

FIG. 2B illustrates a portion of the anesthesia machine 10 and a removable vaporizer 100 fully received into a corresponding interface 11 (not visible) of the anesthesia machine 10, and further illustrating an empty interface 11 configured to receive another vaporizer 100 (not shown), according to embodiments. FIG. 2B further shows the two buttons 210 from the two corresponding engagement mechanisms 200 (not fully shown). The left-side button 210 is in a predetermined position indicating that the corresponding vaporizer 100 is fully received or engaged with the anesthesia machine 10. In the depicted embodiment, the predetermined position is when the outer surface of the left-side button 210 is substantially flush with the housing 14 (e.g., surrounding housing) of the anesthesia machine 10. In the depicted embodiment, the right-side button 210 is also in a predetermined position when a corresponding vaporizer 100 has not been inserted into the interface 11 at all. In the depicted embodiment, the predetermined position for a given button 210 when a corresponding vaporizer 100 has not been inserted into the interface 11 at all is that the outer surface of the given button 210 is substantially flush with the housing 14 (e.g., surrounding housing) of the anesthesia machine 10.

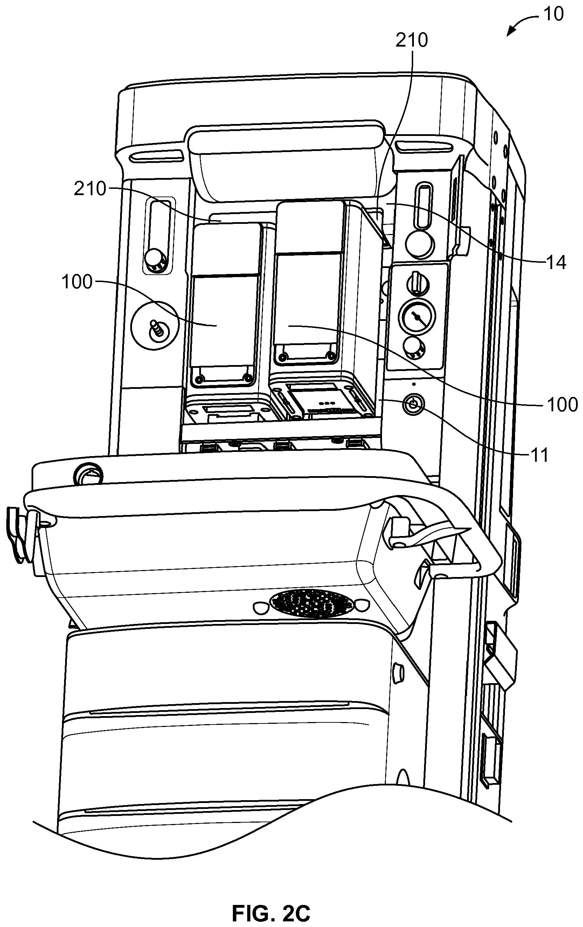

FIG. 2C illustrates a portion of the anesthesia machine 10 and two removable vaporizers 100, with the left-side vaporizer 100 fully received by the left-side interface 11 of the anesthesia machine 10. This arrangement is similar to what is shown with the left-side vaporizer 100 in FIGS. 2A and 2B. Correspondingly, the left-side button 210 is in the predetermined position. FIG. 2C further shows the right-side vaporizer 100 being only partially received by the right-side interface 11 of the anesthesia machine 10. In this embodiment, the outer surface of the button 210 is not in the predetermined position (e.g., it is recessed from the housing 14, e.g., surrounding housing, of the anesthesia machine 10).

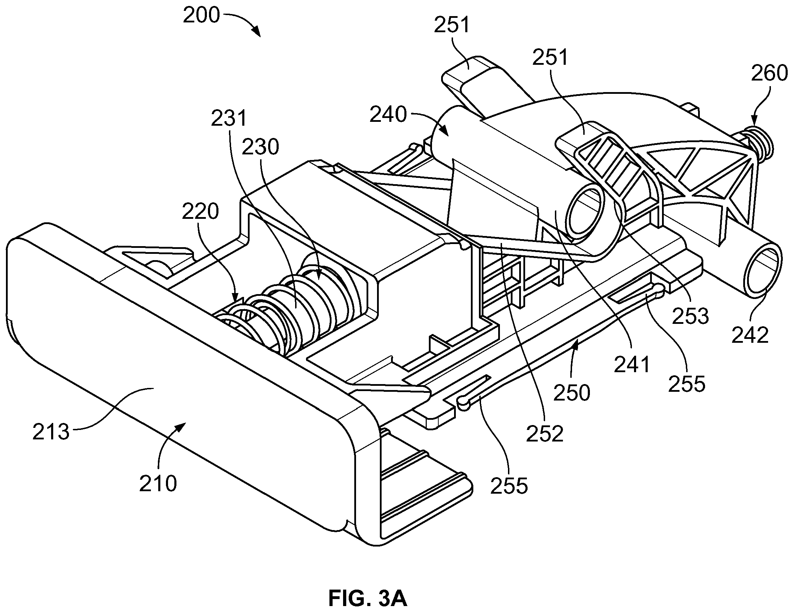

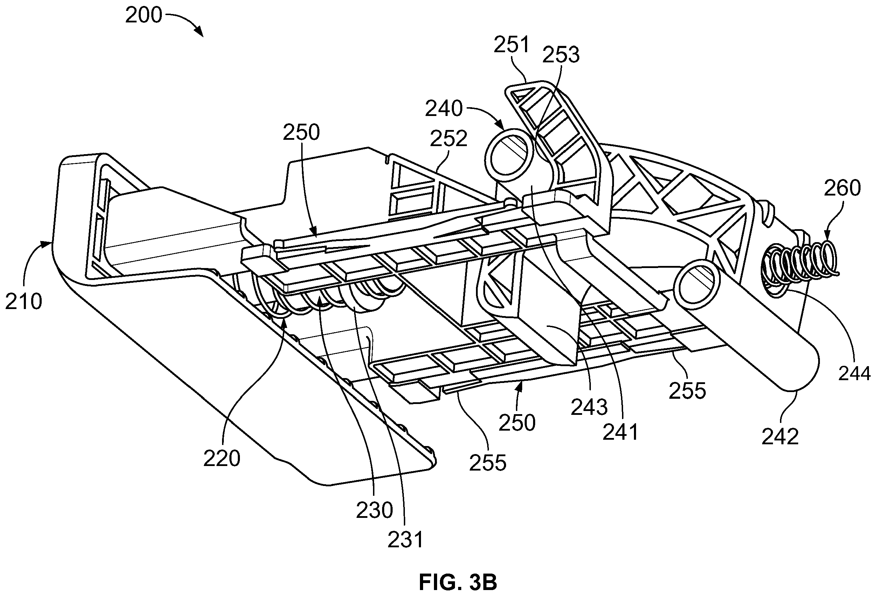

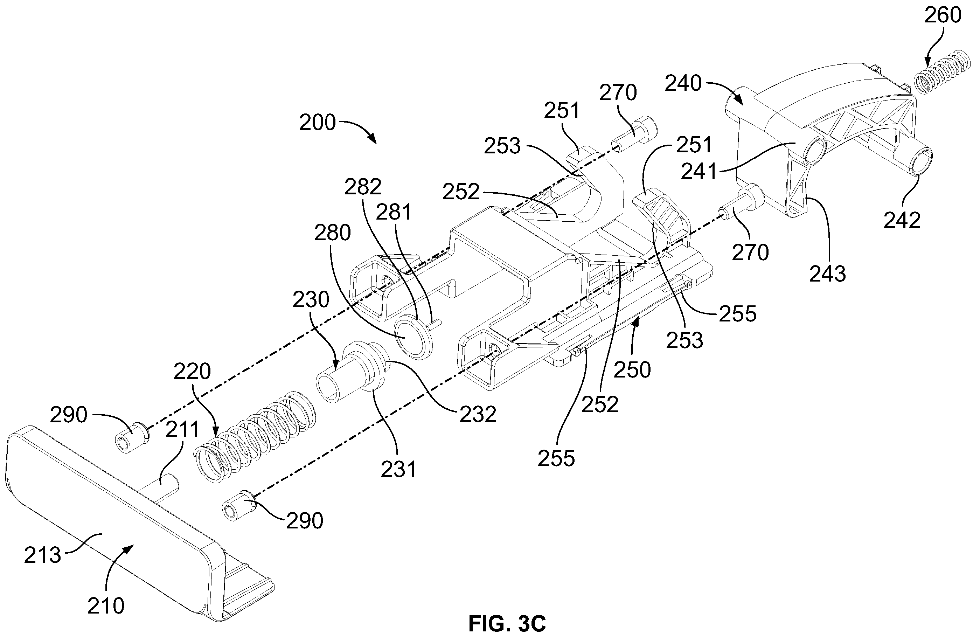

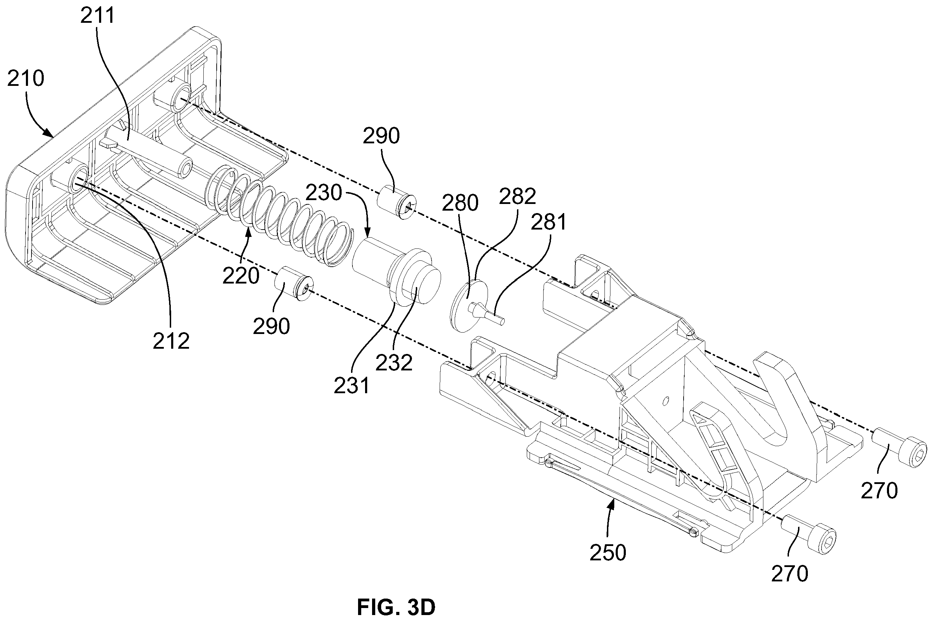

FIGS. 3A, 3B, 3C, and 3D illustrate different views of the engagement mechanism 200 for engaging the vaporizer 100 in the interface 11 of the anesthesia machine 10, according to embodiments. As shown in FIGS. 1, 2A, 2B, and 2C, the anesthesia machine 10 includes or houses two such engagement mechanisms 200, although one, three, or more engagement mechanisms 200 may be included or housed. Particularly, FIG. 3A illustrates a top perspective view of the engagement mechanism 200 for engaging the vaporizer 100 (not shown) in the interface 11 (not shown) of the anesthesia machine 10 (not shown), according to embodiments. FIG. 3B illustrates a bottom perspective view of the engagement mechanism 200 shown in FIG. 3A, according to embodiments. FIG. 3C illustrates an exploded view of the engagement mechanism 200 shown in FIGS. 3A and 3B, according to embodiments. FIG. 3D illustrates an exploded view of a portion of the engagement mechanism 200 shown in FIGS. 3A, 3B, and 3C, according to embodiments.

In the overall embodiment illustrated, engagement mechanism 200 includes a button 210, a button-return spring 220, a sheath 230, a latch 240, a wedge 250, a latch compression spring 260, fasteners 270, a nipple 280, and inserts 290, although more or fewer components may be used according to embodiments.

The button 210 includes an outer surface 213 configured to allow interaction with a user (e.g., interaction with a user's hand or fingers). The button 210 may be an integral piece, as shown, or may be formed by a plurality of coupled portions, such as the different portions described herein. The button 210 may include a shaft 211 and one or more insert-receiving holes 212. The shaft 211 may be cylindrical. The shaft 211 may receive the button-return spring sleeve 230 (e.g., coaxially). The shaft 211 may further receive the button-return spring 220 (e.g., coaxially). The insert-receiving hole(s) 212 may receive the inserts 290.

The button-return spring 220 may be a coil spring or other type of spring. The button-return spring 220 may be formed or include metal or plastic. The button-return spring 220 may be received by the button shaft 211 and/or the sleeve 230. The button-return spring 220 may abut the button 210 and the sleeve (e.g., a collar of the sleeve 230). The button-return spring 220 may be interposed between the button 210 and the sleeve 230 (e.g., a collar 231 of the sleeve 230) and housing 14 of the anesthesia machine 10 (not shown in FIGS. 3A-3D). The button-return spring 220 may be coupled to the button 210 and/or the sleeve 230 (e.g., the collar 231 of the sleeve 230). Instead of the sleeve 230, the button-return spring 220 may couple directly with the housing 14 of the anesthesia machine 10. The button-return spring 220 may couple with the button 210, sleeve 230, and/or housing 14 of the anesthesia machine 10 by virtue of compression of the button-return spring 220 (e.g., a given end of the button-return spring 220 may exert a force against another component).

The sleeve 230 may include a collar 231 and insert 232. The sleeve 230 may be received by or receive the shaft 211 of the button 210. The sleeve 230 may further be received by or receive a portion of the housing 14 of the anesthesia device 10 (e.g., through an aperture in the housing 14). The sleeve 230 may otherwise be coupled to the housing 14. The sleeve 230 may engage with the housing 14 via a sleeve insert 232. The sleeve 230 may not change position substantially during operation of the engagement mechanism 200. The sleeve 230 (and/or the housing 14) may limit an inward movement of the button 210.

The nipple 280 may include an insert 281 and a pad 282. The nipple 280 may be an integral piece or a collection of smaller portions coupled together, such as the portions described herein. The insert 281 may be inserted into an aperture in the wedge 250 to secure the nipple 280 to the wedge 250. The pad 282 may extend from the insert 281 and face towards the sheath 230.

The latch 240 may include one or more laterally projecting portions 241, a pivot 242, an engagement portion 243, and a latch spring aperture 244, as shown. The latch 240 may be one integral piece or be formed from a collection of different portions, such as the specific portions described herein. The latch 240 may include a lattice as shown to reduce the weight and expense of the latch 240. The laterally projecting portion(s) 241 may be round (e.g., circular or cylindrical. the laterally projecting portion(s) 241 may have a hollow interior region. The laterally projecting portion(s) 241 may engage or interact with the wedge 250. The pivot 242 may interface with the housing 14 of the anesthesia machine 10 (either by abutting the housing 14 or indirectly interfacing with the housing). The latch 240 may rotate about the pivot 242. The engagement portion 243 may project downwardly as shown, or may project in another direction. The engagement portion 243 may selectively engage with a vaporizer 100. The engagement portion 243 may include a substantially rounded side and a substantially vertical side. The latch spring aperture 244 may receive the latch spring 260, such that a portion of the latch spring 260 is contained within the latch 240.

The wedge 250 may include one or more hook(s) 251, one or more upwardly-facing sloped region(s) 252, one or more downwardly-facing sloped region(s) 253, one or more fastener-receiving apertures 254, and one or more wedge housing engagement portions 255, according to the embodiment depicted. The wedge 250 may be an integral piece, as shown, or may be formed by a plurality of coupled portions, such as the different portions described herein. There may be two hooks 251, one on the right side and one on the left. The hook(s) 251 may be elongated and extend along the dimension along which the wedge 250 travels. Each hook 251 may have an upwardly-facing sloped region 252 and a downwardly-facing sloped region 253. The upwardly-facing sloped region 252 and the downwardly-facing sloped region 253 may extend towards each other into a U-shaped region or V-shaped region that connects the two. The fastener-receiving aperture(s) 254 may receive corresponding fastener(s) 270. The wedge housing engagement portion(s) 255 may engage with the housing 14 (not shown) to slide inwardly and outwardly along the housing 14 while being constrained from movement in other directions. The wedge housing engagement portion(s) 255 may include rails as shown, which are received by tracks in the housing 14 (not shown). The wedge housing engagement portion(s) 255 may include spring arms as shown, which engage with the housing 14 to stabilize the wedge 250 as it travels inwardly and outwardly in the anesthesia machine 10. The wedge housing engagement portion(s) 255 may further have lateral recess(es) to reduce the amount of surface area on the wedge housing engagement portion(s) 255 that contacts the housing 14.

The latch compression spring 260 may be a coil spring or other type of spring. The latch compression spring 260 may be formed or include metal or plastic. The latch compression spring 260 may engage or couple with the housing 14 on one end (e.g., directly or indirectly engage or couple with the housing 14). The latch compression spring 260 on the other side may extend through the latch spring aperture 244 in the latch 240 and may engage or couple with the latch 240 (e.g., directly or indirectly engage or couple the latch 240). The latch compression spring 260 may engage or couple with the housing 14 and latch 240 by virtue of compression of the latch compression spring 260 (e.g., a given end of the latch compression spring 260 may exert a force against another component).

The fastener(s) 270 may be bolts or screws or other types of fasteners. The fastener(s) 270 may couple (e.g., statically couple) the button 210 and the wedge 250. The fastener(s) may extend through the fastener-receiving aperture(s) 254 in the wedge 250 and be received by the button hole insert(s) 290 located in the insert-receiving hole(s) 212 in the button 210.

The engagement mechanism 200 may be configured as follows. The shaft 211 of the button 210 may receive a hollow interior region of the sleeve 230. The button-return spring 220 may extend over the shaft 211 of the button 210 and the sleeve 230. The button-return spring 220 may always remain compressed to a degree such that it always exerts forces against the button 210 and the sleeve 230 (e.g., the collar 231 of the sleeve 230). The insert 232 of the sleeve 230 may be inserted into an aperture in the housing 14 of the anesthesia machine 10. The inserts 280 may be received by the insert-receiving apertures 212 in the button 210. The fasteners 270 may extend through the faster-receiving apertures 254 and received by the inserts 280 to couple (e.g., statically couple) the button 210 with the wedge 250. The nipple 280 may engage with the wedge 250 via the nipple insert 281 such that the nipple 280 moves with the wedge 250. The laterally-extending portions 241 of the latch 240 may be positioned in or on corresponding hooks 251 in the wedge 250. The wedge 250 may be moveable inwardly and outwardly along the housing 14 via the wedge housing engagement portions 255 and corresponding features on the housing 14. The pivot 242 of the latch 240 may press against the housing 14, such that the latch 240 rotates around the point where the pivot 242 presses against the housing 14. The latch compression spring 260 may engage with the latch 260 through the latch spring aperture 244 and with the housing 14.

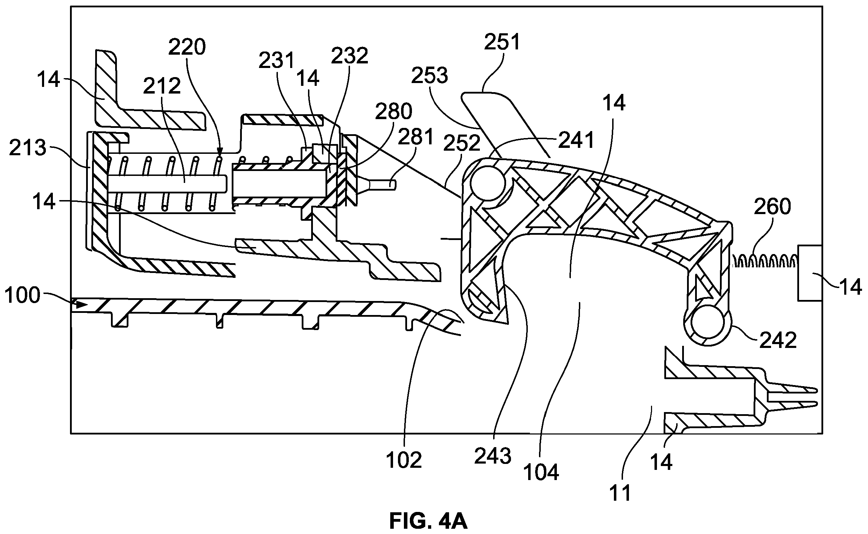

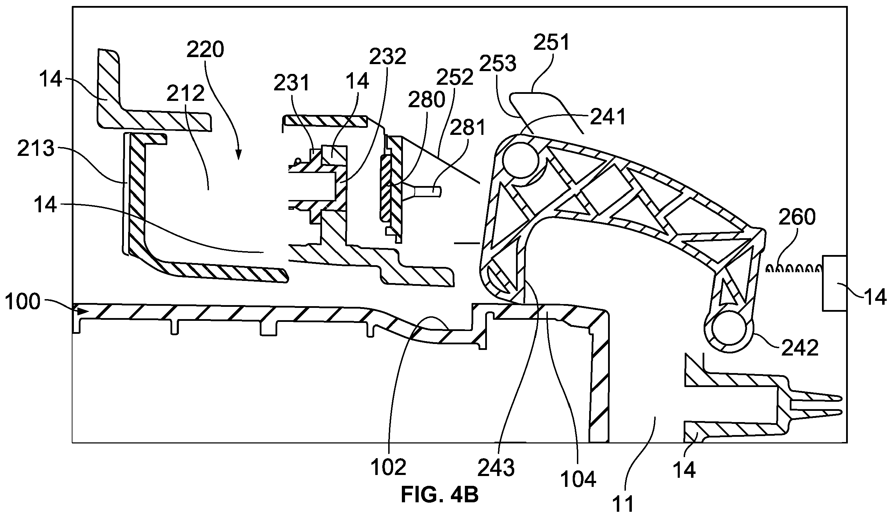

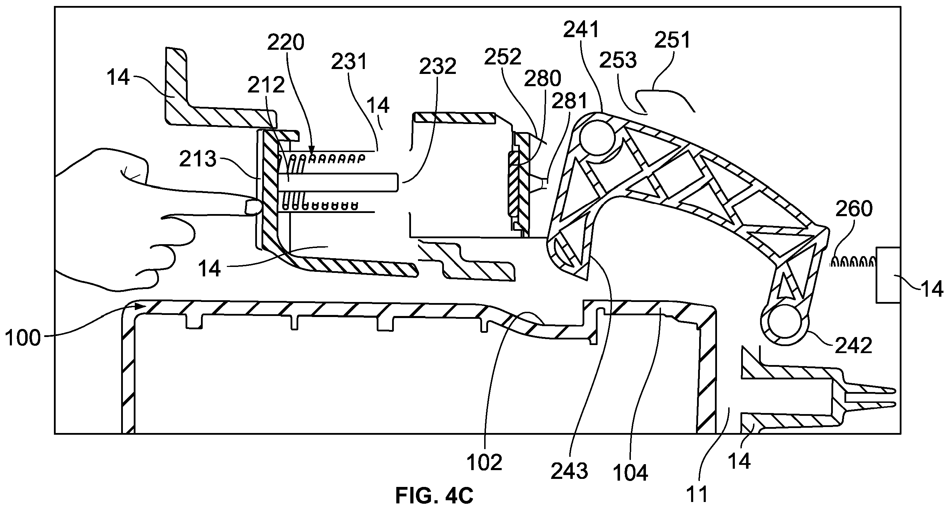

FIGS. 4A, 4B, and 4C show different modes of operation of the engagement mechanism 200 with respect to the housing 14 of the anesthesia machine 10 and the vaporizer 100, according to embodiments. FIG. 4A illustrates a cross-sectional view of a portion of an anesthesia machine 10 including the engagement mechanism 200, and a portion of a removable vaporizer 100, where the vaporizer 100 is fully inserted into an interface 11 of the anesthesia machine 10, according to embodiments. FIG. 4B illustrates a cross-sectional view of a portion of the anesthesia machine 10 including the engagement mechanism 200, and a portion of the vaporizer 100, where the vaporizer 100 is partially inserted into the interface 11 of the anesthesia machine 10, according to embodiments. FIG. 4C illustrates a cross-sectional view of a portion of the anesthesia machine 10 including the engagement mechanism 200, and a portion of the vaporizer 100, where the vaporizer 100 is fully inserted into the interface 11 of the anesthesia machine 10, and where a user has interacted with the engagement mechanism 200 to disengage the engagement mechanism 200 from the vaporizer 100, according to embodiments. FIGS. 4A, 4B, and 4C show only one hook 251 in the wedge 250, but it is understood that a similar arrangement may be optionally included for another hook 251, as depicted in FIGS. 3A-3D.

As shown in FIG. 4A, the vaporizer 100 may include a wall having an outer surface. The outer surface forms a recess 102 and a shelf 104. When the vaporizer 100 has been fully received by the interface 11 of the anesthesia machine 10, the engagement portion 243 of the latch 240 may extend into the recess 102 of the vaporizer 100. A force may be exerted by the latch compression spring 260 such that the engagement portion 243 presses downwardly into the recess against the vaporizer. If there is no force exerted, the engagement portion 243 of the latch 240 may still extend into the recess 102 of the vaporizer 100. The shape of the engagement portion 243 of the latch 240 may assist in securing the vaporizer 100 in the interface 11 of the anesthesia machine 10. As shown, the engagement portion 243 of the latch may only abut the recess 102 of the vaporizer 100 at a limited surface area. If a pulling force is exerted on the vaporizer 100, the shape of the recess 102 and the shape of the engagement portion 243 may prevent the engagement portion 243 from moving upwardly, in addition to the effect of a downward force being applied to the engagement portion 243.

As shown in FIG. 4A, the latch compression spring 260 may be compressed, but less compressed than in FIGS. 4B and 4C. The latch 240 may be rotated counterclockwise around the location where the pivot 242 abuts the housing 14. The laterally extending portion 241 of the latch 240 may extend into corresponding U-shaped region in the hook 251. The laterally extending portion 241 may be fully received by the hook 251 of the wedge 250. The position of the laterally extending portion 241 with respect to the hook 251 may determine the location of the wedge 250. When the button 210 is coupled to the wedge 250 (e.g., statically coupled), the position of the button 210 may further be determined with respect to the position of the wedge 250. In the arrangement of FIG. 4A, the outer surface 213 of the button 210 may be at a predetermined location. In this embodiment, the predetermined location of the outer surface 213 of the button 210 may be substantially flush with respect to the outer surface of the housing 14 of the anesthesia machine 10 proximate the button 210. When the outer surface 213 of the button 210 is in the predetermined position, this indicates that the vaporizer 100 may be fully received by the interface 11 of the anesthesia machine 10 and/or that the engagement mechanism 200 may be engaged the vaporizer 100. The button return spring 220 may be uncompressed to its maximum extent, thereby maintaining the outer surface 213 of the button 210 in the predetermined position.

As shown in FIG. 4B, when the vaporizer 100 is not fully received by the interface 11 of the anesthesia machine 10, the engagement portion 243 of the latch 240 may be forced upwardly by the shelf 104 of the vaporizer 100. This may cause the latch 240 to rotate clockwise around the point where the pivot 242 abuts the housing 14. This may further cause the latch compression spring 260 to compress further, and may cause the laterally extending portion 241 of the latch 240 to engage with the downwardly-facing sloped region 253 of the wedge 250. As the laterally extending portion 241 presses against the downwardly-facing sloped region 253 of the wedge 250, this may force the wedge 250 to move inwardly, thereby causing the button 210 to be pulled inwardly. When the button 210 is pulled inwardly, the outer surface 213 of the button 210 may be moved out of the predetermined position. This may indicate that the vaporizer 100 has not been fully received by the interface 11 of the anesthesia machine 10. This may further compress the button-return spring 220, causing relatively stronger forces to be exerted against the button 210 and the housing 14 via the sleeve 230.

As shown in FIG. 4C, when the outer surface 213 of the button 210 is engaged (e.g., by a user's finger or hand) and forced inwardly, this may cause the button 210 to move inwardly, as well as the wedge 250 to move inwardly as well. This inward movement may also compress the button-return spring 220, such that the button 210 may be forced outwardly once the engagement force is removed from the outer surface 213 of the button 210. As the wedge 250 is moved inwardly, the laterally projecting portion 241 of the latch 240 may engage with the upwardly-facing sloped region 252 on the hook 251 of the wedge 250. As the wedge 250 moves farther inwardly, the latch 240 may rotate clockwise around the location where the pivot 242 abuts the housing 14. This may cause the latch engagement portion 243 of the latch 240 to be repositioned such that it is no longer within the recess 102 of the vaporizer 100. Once the latch engagement portion 243 of the latch 240 has been removed from the recess 102 of the vaporizer 100, the vaporizer 100 may be removed from the interface 11 of the anesthesia machine 10.

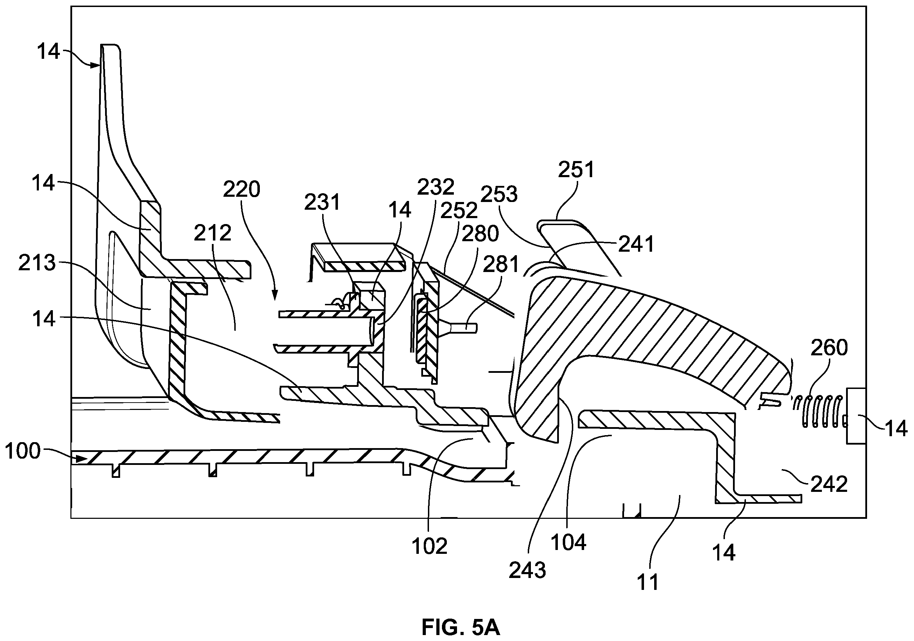

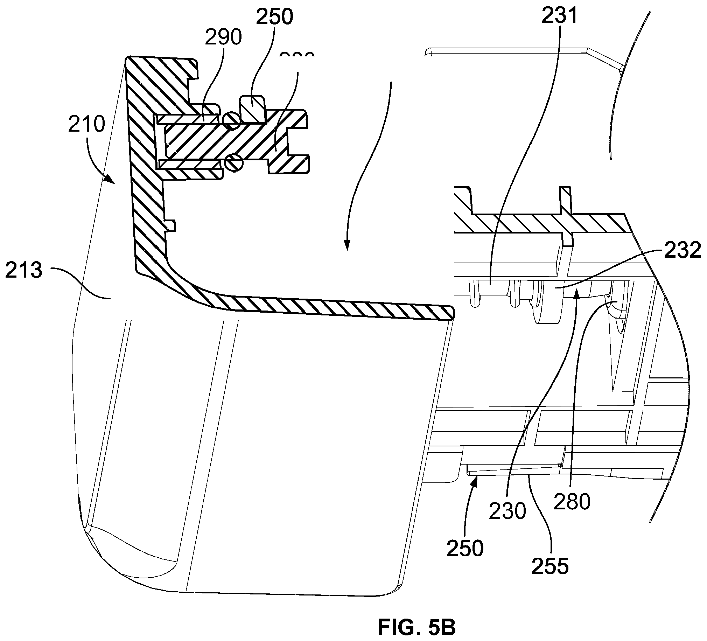

FIG. 5A is similar to FIG. 4B, except that the system is shown with perspective to provide additional context about the arrangement of the anesthesia machine 10, the engagement mechanism 200, and the vaporizer 100. FIG. 5B illustrates a cross-sectional view along the plane of the right-side fastener 270 to show the arrangement of the button 210, the wedge 250, the housing 14, the spring 220, and the nipple 280.

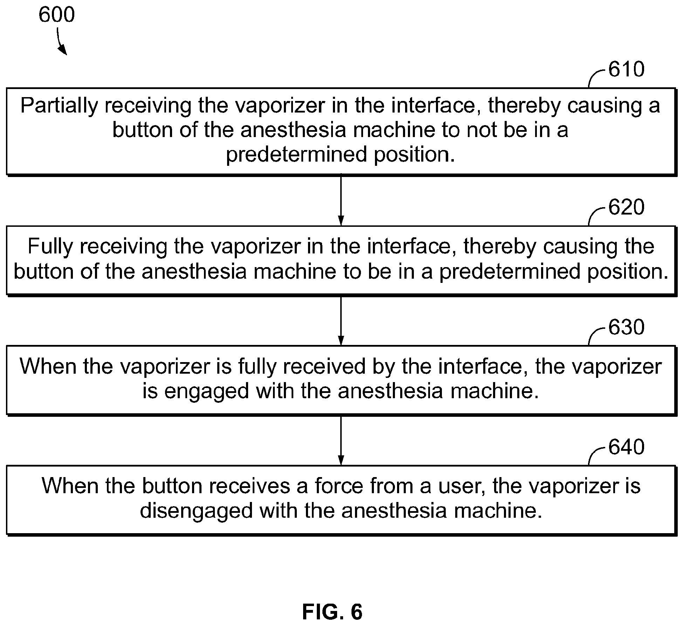

FIG. 6 shows a flowchart 600 of a method for engaging and disengaging a vaporizer 100 from an anesthesia machine 10, according to embodiments. The method is described in context of vaporizer 100 and anesthesia machine 10 (including the engagement mechanism 200 and the button 210), but the method is not so limited. At step 610, the vaporizer 100 is partially received at the interface 11 of the anesthesia machine 10. This causes the button 210 of the engagement mechanism 200 of the anesthesia machine 10 to not be in a predetermined position (e.g., a predetermined position with respect to the housing 14 of the anesthesia machine 10. FIG. 4B is an illustrative embodiment. At step 620, the vaporizer 100 is fully received by the interface 11 of the anesthesia machine 10. This causes the button 210 of the engagement mechanism of the anesthesia machine 10 to be in the predetermined position. FIG. 4A is an illustrative embodiment. At step 630, when the vaporizer 100 is fully received by the interface 11 of the anesthesia machine 10, the vaporizer 100 is engaged by the anesthesia machine 10. The vaporizer 100 is engaged such that it cannot be non-destructively removed from the anesthesia machine 10 or otherwise moved out of place in the interface 11 of the anesthesia machine 10. FIG. 4A is an illustrative embodiment. At step 640, the button 210 receives a force from a user (e.g., a force applied to the outer surface 213 of the button 210), thereby causing the vaporizer 100 to be disengaged from the anesthesia machine 10, such that the vaporizer 100 can be removed (e.g., removed by the user) from the anesthesia machine 10 or otherwise moved within the interface 11 of the anesthesia machine 10. FIG. 4C is an illustrative embodiment.

**Parts List**

| **Part Name** | **Reference** |
|---|---|
| Anesthesia machine | 10 |
| Anesthesia machine interface | 11 |
| Anesthesia machine housing | 14 |
| Vaporizer | 100 |
| Vaporizer upper surface recess | 102 |
| Vaporizer upper surface shelf | 104 |
| Engagement mechanism | 200 |
| Button | 210 |
| Button shaft | 211 |
| Button insert-receiving apertures | 212 |
| Button outer surface | 213 |
| Button-return spring | 220 |
| Sleeve | 230 |
| Sleeve collar | 231 |
| Sleeve insert | 232 |
| Latch | 240 |
| Latch lateral portions | 241 |
| Latch pivot | 242 |
| Latch engagement portion | 243 |
| Latch spring aperture | 244 |
| Wedge | 250 |
| Wedge hooks | 251 |
| Wedge upwardly-facing sloped regions | 252 |
| Wedge downwardly-facing sloped regions | 253 |
| Wedge fastener-receiving apertures | 254 |
| Wedge housing engagement portions | 255 |
| Latch compression spring | 260 |
| Fasteners | 270 |
| Nipple | 280 |
| Nipple insert | 281 |
| Nipple pad | 282 |
| Inserts | 290 |
| Flow chart | 300 |

## Claims

1. An anesthesia machine (10) for engaging a vaporizer (100) with an interface (11) of the anesthesia machine, wherein the vaporizer includes an upper surface with a shelf (104) and a recess (102), the anesthesia machine comprising:
a button (210) configured to receive an engagement force from a user and responsively move inwardly;
a wedge (250) including at least one upwardly-facing sloped region (252) and at least one downwardly-facing sloped region (253); and
a latch (240) including an engagement portion (243) configured to engage with the upper surface of the vaporizer, and including at least one laterally-projecting portion (241) configured to engage with the at least one upwardly-facing sloped region and the at least one downwardly-facing sloped region of the wedge,
wherein the engagement portion of the latch is configured to be forced upwardly when the vaporizer is being inserted into the interface of the anesthesia machine in response to the engagement portion of the latch engaging with the shelf of the upper surface of the vaporizer, and wherein the at least one laterally-projecting portion of the latch is configured to engage with the at least one downwardly-facing sloped region of the wedge when the engagement portion of the latch engages with the shelf of the upper surface of the vaporizer, thereby causing the wedge and the button to move inwardly,
wherein the engagement portion of the latch is configured to be received by the recess of the upper surface of the vaporizer, thereby causing the wedge and the button to move outwardly, and
wherein the button is configured to receive the engagement force while the engagement portion of the latch is received in the recess in the upper surface of the vaporizer, thereby causing the at least one upwardly-facing sloped region of the wedge to engage with the at least one laterally-projecting portion of the latch, thereby causing the engagement portion of the latch to be lifted upwardly out of the recess in the upper surface of the vaporizer.

2. The anesthesia machine of claim 1, further comprising a button return spring (220) interposed between the button and a housing (14) of the anesthesia machine, wherein the button return spring causes the button and the wedge to move outwardly.

3. The anesthesia machine of claim 1, further comprising a latch compression spring (260) configured to exert a force on the latch thereby causing the engagement portion of the latch to exert a force downwardly.

4. The anesthesia machine of claim 1, wherein the latch comprises a pivot (242) configured to be rotatably moved with respect to the housing, such that the latch is configured to rotate around the pivot.

5. The anesthesia machine of claim 1, wherein the interface of the anesthesia machine comprises a housing having an outer surface, wherein when an outer surface of the button is flush with the outer surface of the housing, it indicates that the vaporizer has been fully received by the interface, and wherein when the outer surface of the button is not flush with the outer surface of the housing, it indicates that the vaporizer has not been fully received by the interface.

6. The anesthesia machine of claim 1, wherein:
the at least one upwardly-facing sloped region of the wedge includes a first upwardly-facing sloped region and a second upwardly-facing sloped region;
the at least one downwardly-facing sloped region of the wedge includes a first downwardly-facing sloped region and a second downwardly-facing sloped region;
the at least one laterally-projecting portion of the latch includes a first laterally-projecting portion and a second laterally-projecting portion;
wherein the first laterally-projecting portion of the latch is configured to engage with the first upwardly-facing sloped region of the wedge and the first downwardly-facing sloped region of the wedge; and
wherein the second laterally-projecting portion of the latch is configured to engage with the second upwardly-facing sloped region of the wedge and the second downwardly-facing sloped region of the wedge.

7. The anesthesia machine of claim 1, wherein a slope of the at least one downwardly-facing sloped region is greater than a slope of the at least one upwardly-facing sloped region.

8. The anesthesia machine of claim 1, wherein the vaporizer comprises a vaporizer configured to contain a gas.

9. The anesthesia machine of claim 1, wherein the wedge includes a hook including one of the at least one downwardly-facing sloped region and one of the at least one upwardly-facing sloped region.

10. The anesthesia machine of claim 1, wherein the wedge comprises a plurality of rails configured to be received by a corresponding plurality of tracks in the housing of the anesthesia machine, and wherein the wedge further comprises a plurality of spring arms configured to secure the wedge to the plurality of tracks.

11. A method for inserting a vaporizer (100) into an interface (11) of an anesthesia machine (10) of any of claims 1 to 10, comprising:
partially receiving the vaporizer in the interface, thereby causing a button (210) of the anesthesia machine to not be in a predetermined position; and
fully receiving the vaporizer in the interface, thereby causing the button of the anesthesia machine to be in a predetermined position.

12. The method of claim 11, wherein when the vaporizer is fully received by the interface, the vaporizer is engaged with the anesthesia machine.

13. The method of claim 12, wherein when the button receives a force from a user, the vaporizer is disengaged with the anesthesia machine.

## Patentansprüche

1. Anästhesiegerät (10) zum Ineingriffbringen eines Verdampfers (100) mit einer Schnittstelle (11) des Anästhesiegeräts, wobei der Verdampfer eine Oberseite mit einer Ablage (104) und einer Aussparung (102) enthält, wobei das Anästhesiegerät Folgendes umfasst:
eine Taste (210), die konfiguriert ist, eine Eingriffskraft von einem Anwender zu empfangen und sich in Reaktion nach innen zu bewegen;
einen Keil (250), der wenigstens einen nach oben gerichteten geneigten Bereich (252) und wenigstens einen nach unten gerichteten geneigten Bereich (253) enthält; und
einen Riegel (240), der einen Eingriffabschnitt (243) enthält, der konfiguriert ist, in die Oberseite des Verdampfers einzugreifen, und der wenigstens einen seitlich vorstehenden Abschnitt (241) enthält, der konfiguriert ist, in den wenigstens einen nach oben gerichteten geneigten Bereich und den wenigstens einen nach unten gerichteten geneigten Bereich des Keils einzugreifen,
wobei der Eingriffabschnitt des Riegels konfiguriert ist, nach oben gezwungen zu werden, wenn der Verdampfer in die Schnittstelle des Anästhesiegeräts in Reaktion darauf eingesetzt wird, dass der Eingriffabschnitt des Riegels in die Ablage der Oberseite des Verdampfers eingreift, und wobei der wenigstens eine seitlich vorstehende Abschnitt des Riegels konfiguriert ist, in den wenigstens einen nach unten gerichteten geneigten Bereich des Keils einzugreifen, wenn der Eingriffabschnitt des Riegels in die Ablage der Oberseite des Verdampfers eingreift, wodurch bewirkt wird, dass sich der Keil und die Taste nach innen bewegen,
wobei der Eingriffabschnitt des Riegels konfiguriert ist, durch die Aussparung der Oberseite des Verdampfers aufgenommen zu werden, wodurch bewirkt wird, dass sich der Keil und die Taste nach außen bewegen, und
wobei die Taste konfiguriert ist, die Eingriffskraft zu empfangen, während der Eingriffabschnitt des Riegels in der Aussparung in der Oberseite des Verdampfers aufgenommen wird, wodurch bewirkt wird, dass der wenigstens eine nach oben gerichtete geneigte Bereich des Keils in den wenigstens einen seitlich vorstehenden Abschnitt des Riegels eingreift, wodurch bewirkt wird, dass der Eingriffabschnitt des Riegels nach oben aus der Aussparung in der Oberseite des Verdampfers gehoben wird.

2. Anästhesiegerät nach Anspruch 1, das ferner eine Tastenrückstellfeder (220) umfasst, die zwischen der Taste und einem Gehäuse (14) des Anästhesiegeräts eingefügt ist, wobei die Tastenrückstellfeder bewirkt, dass sich die Taste und der Keil nach außen bewegen.

3. Anästhesiegerät nach Anspruch 1, das ferner eine Riegeldruckfeder (260) umfasst, die konfiguriert ist, eine Kraft auf den Riegel auszuüben, wodurch bewirkt wird, dass der Eingriffabschnitt des Riegels eine Kraft nach unten ausübt.

4. Anästhesiegerät nach Anspruch 1, wobei der Riegel einen Drehzapfen (242) umfasst, der konfiguriert ist, in Bezug auf das Gehäuse drehbar bewegt zu werden, so dass der Riegel konfiguriert ist, sich um den Drehzapfen zu drehen.

5. Anästhesiegerät nach Anspruch 1, wobei die Schnittstelle des Anästhesiegeräts ein Gehäuse mit einer Außenfläche umfasst, wobei dann, wenn eine Außenfläche der Taste mit der Außenfläche des Gehäuses bündig ist, dies angibt, dass der Verdampfer vollständig durch die Schnittstelle aufgenommen worden ist, und wobei dann, wenn die Außenfläche der Taste nicht mit der Außenfläche des Gehäuses bündig ist, dies angibt, dass der Verdampfer nicht vollständig durch die Schnittstelle aufgenommen worden ist.

6. Anästhesiegerät nach Anspruch 1, wobei:
der wenigstens eine nach oben gerichtete geneigte Bereich des Keils einen ersten nach oben gerichteten geneigten Bereich und einen zweiten nach oben gerichteten geneigten Bereich enthält;
der wenigstens eine nach unten gerichtete geneigte Bereich des Keils einen ersten nach unten gerichteten geneigten Bereich und einen zweiten nach unten gerichteten geneigten Bereich enthält;
der wenigstens eine seitlich vorstehende Abschnitt des Riegels einen ersten seitlich vorstehenden Abschnitt und einen zweiten seitlich vorstehenden Abschnitt enthält;
wobei der erste seitlich vorstehende Abschnitt des Riegels konfiguriert ist, in den ersten nach oben gerichteten geneigten Bereich des Keils und den ersten nach unten gerichteten geneigten Bereich des Keils einzugreifen; und
wobei der zweite seitlich vorstehende Abschnitt des Riegels konfiguriert ist, in den zweiten nach oben gerichteten geneigten Bereich des Keils und den zweiten nach unten gerichteten geneigten Bereich des Keils einzugreifen.

7. Anästhesiegerät nach Anspruch 1, wobei eine Neigung des wenigstens einen nach unten gerichteten geneigten Bereichs größer als eine Neigung des wenigstens einen nach oben gerichteten geneigten Bereichs ist.

8. Anästhesiegerät nach Anspruch 1, wobei der Verdampfer einen Verdampfer umfasst, der konfiguriert ist, ein Gas zu enthalten.

9. Anästhesiegerät nach Anspruch 1, wobei der Keil einen Haken enthält, der einen des wenigstens einen nach unten gerichteten geneigten Bereichs und einen des wenigstens einen nach oben gerichteten geneigten Bereichs enthält.

10. Anästhesiegerät nach Anspruch 1, wobei der Keil mehrere Schienen umfasst, die konfiguriert sind, durch entsprechende mehrere Bahnen im Gehäuse des Anästhesiegeräts aufgenommen zu werden, und wobei der Keil ferner mehrere Federarme umfasst, die konfiguriert sind, den Keil an den mehreren Bahnen zu befestigen.

11. Verfahren zum Einsetzen eines Verdampfers (100) in eine Schnittstelle (11) eines Anästhesiegeräts (10) nach einem der Ansprüche 1 bis 10, wobei das Verfahren Folgendes umfasst:
teilweises Aufnehmen des Verdampfers in der Schnittstelle, wodurch bewirkt wird, dass sich eine Taste (210) des Anästhesiegeräts nicht an einer vorgegebenen Position befindet; und
vollständiges Aufnehmen des Verdampfers in der Schnittstelle, wodurch bewirkt wird, dass sich die Taste des Anästhesiegeräts an einer vorgegebenen Position befindet.

12. Verfahren nach Anspruch 11, wobei dann, wenn der Verdampfer vollständig durch die Schnittstelle aufgenommen worden ist, der Verdampfer in das Anästhesiegerät eingreift.

13. Verfahren nach Anspruch 12, wobei dann, wenn die Taste eine Kraft von einem Anwender empfängt, der Verdampfer aus dem Anästhesiegerät gelöst wird.

## Revendications

1. Machine d'anesthésie (10) permettant d'engager un vaporisateur (100) avec une interface (11) de la machine d'anesthésie, le vaporisateur comprenant une surface supérieure avec une saillie (104) et un renfoncement (102), la machine d'anesthésie comprenant :
un bouton (210) configuré pour recevoir une force d'engagement d'un utilisateur et se déplacer en réponse vers l'intérieur ;
un coin (250) comprenant au moins une région inclinée tournée vers le haut (252) et au moins une région inclinée tournée vers le bas (253) ; et
un loquet (240) comprenant une partie d'engagement (243) configurée pour venir en prise avec la surface supérieure du vaporisateur, et comprenant au moins une partie faisant saillie latéralement (241) configurée pour venir en prise avec l'au moins une région inclinée tournée vers le haut et l'au moins une région inclinée tournée vers le bas du coin,
la partie d'engagement du loquet étant configurée pour être forcée vers le haut lorsque le vaporisateur est inséré dans l'interface de la machine d'anesthésie en réponse à la partie d'engagement du loquet venant en prise avec la saillie de la surface supérieure du vaporisateur, et l'au moins une partie faisant saillie latéralement du loquet étant configurée pour venir en prise avec l'au moins une région inclinée tournée vers le bas du coin lorsque la partie d'engagement du loquet vient en prise avec la saillie de la surface supérieure du vaporisateur, amenant ainsi le bouton et le coin à se déplacer vers l'intérieur,
la partie d'engagement du loquet étant configurée pour être reçue par l'évidement de la surface supérieure du vaporisateur, amenant ainsi le bouton et le coin à se déplacer vers l'extérieur, et
le bouton étant configuré pour recevoir la force d'engagement tandis que la partie d'engagement du loquet est reçue dans l'évidement de la surface supérieure du vaporisateur, amenant ainsi l'au moins une région inclinée tournée vers le haut du coin à venir en prise avec l'au moins une partie faisant saillie latéralement du loquet, amenant ainsi la partie d'engagement du loquet à être soulevée vers le haut hors de l'évidement de la surface supérieure du vaporisateur.

2. Machine d'anesthésie selon la revendication 1, comprenant en outre un ressort de rappel de bouton (220) interposé entre le bouton et un logement (14) de la machine d'anesthésie, le ressort de rappel de bouton amenant le bouton et le coin à se déplacer vers l'extérieur.

3. Machine d'anesthésie selon la revendication 1, comprenant en outre un ressort de compression de loquet (260) configuré pour exercer une force sur le loquet, amenant ainsi la partie d'engagement du loquet à exercer une force vers le bas.

4. Machine d'anesthésie selon la revendication 1, le loquet comprenant un pivot (242) configuré pour être déplacé de manière rotative par rapport au logement, de sorte que le loquet est configuré pour tourner autour du pivot.

5. Machine d'anesthésie selon la revendication 1, l'interface de la machine d'anesthésie comprenant un logement ayant une surface extérieure, lorsqu'une surface extérieure du bouton affleure la surface extérieure du logement, cela indique que le vaporisateur a été entièrement reçu par l'interface, et lorsque la surface extérieure du bouton n'affleure pas la surface extérieure du logement, cela indique que le vaporisateur n'est pas entièrement reçu par l'interface.

6. Machine d'anesthésie selon la revendication 1,
l'au moins une région inclinée tournée vers le haut du coin comprenant une première région inclinée tournée vers le haut et une seconde région inclinée tournée vers le haut ;
l'au moins une région inclinée tournée vers le bas du coin comprenant une première région inclinée tournée vers le bas et une seconde région inclinée tournée vers le bas ;
l'au moins une partie faisant saillie latéralement du loquet comprenant une première partie faisant saillie latéralement et une deuxième partie faisant saillie latéralement ;
la première partie faisant saillie latéralement du loquet étant configurée pour venir en prise avec la première région inclinée tournée vers le haut du coin et la première région inclinée tournée vers le bas du coin ; et
la seconde partie faisant saillie latéralement du loquet étant configurée pour venir en prise avec la seconde région inclinée tournée vers le haut du coin et la seconde région inclinée tournée vers le bas du coin.

7. Machine d'anesthésie selon la revendication 1, une pente de l'au moins une région inclinée tournée vers le bas étant supérieure à une pente de l'au moins une région inclinée tournée vers le haut.

8. Machine d'anesthésie selon la revendication 1, le vaporisateur comprenant un vaporisateur configuré pour contenir un gaz.

9. Machine d'anesthésie selon la revendication 1, le coin comprenant un crochet comprenant l'une de l'au moins une région inclinée tournée vers le bas et l'une de l'au moins une région inclinée tournée vers le haut.

10. Machine d'anesthésie selon la revendication 1, le coin comprenant une pluralité de rails configurés pour être reçus par une pluralité de pistes correspondantes dans le logement de la machine d'anesthésie, et le coin comprenant en outre une pluralité de bras de ressort configurés pour fixer le coin à la pluralité de pistes.

11. Procédé d'insertion d'un vaporisateur (100) dans une interface (11) d'une machine d'anesthésie (10) selon l'une quelconque des revendications 1 à 10, comprenant : la réception partielle du vaporisateur dans l'interface, amenant ainsi un bouton (210) de la machine d'anesthésie à ne pas se trouver dans une position prédéterminée ; et la réception complète du vaporisateur dans l'interface, amenant ainsi le bouton de la machine d'anesthésie à se trouver dans une position prédéterminée.

12. Procédé selon la revendication 11, lorsque le vaporisateur est entièrement reçu par l'interface, le vaporisateur étant en prise avec la machine d'anesthésie.

13. Procédé selon la revendication 12, lorsque le bouton reçoit une force d'un utilisateur, le vaporisateur étant désengagé de la machine d'anesthésie.
